# EUROPEAN PATENT APPLICATION

(11) **EP 1 559 320 A1**
(43) Date of publication of application: **03.08.2005**
(21) Application number: 03759002.3
(22) Date of filing: 28.10.2003
(51) Int. Cl.: A01N 43/40, C07D 213/61, C07D 213/64, C07D 213/65, C07D 213/69, C07D 213/70

(54) **3-BENZOYL-2,4,5-SUBSTITUTED PYRIDINE DERIVATIVES OR SALTS THEREOF AND BACTERICIDES CONTAINING THE SAME**

(30) Priority: 31.10.2002 JP 2002317759
(71) Applicant: ISHIHARA SANGYO KAISHA, LTD., Nishi-ku, Osaka 550-0002 (JP)
(72) Inventor: NISHIDE, Hisaya, c/o Ishihara Sangyo Kaisha Ltd., Kusatsu-shi, Shiga 525-0025 (JP); OGAWA, Munekazu, c/o Ishihara Sangyo Kaisha Ltd., Kusatsu-shi, Shiga 525-0025 (JP); TANIMURA, Toyoshi, c/o Ishihara Sangyo Kaisha Ltd., Kusatsu-shi, Shiga 525-0025 (JP); HIGUCHI, Koji, c/o Ishihara Sangyo Kaisha Ltd., Kusatsu-shi, Shiga 525-0025 (JP); KOMINAMI, Hidemasa, c/o Ishihara Sangyo Kaisha Ltd, Kusatsu-shi, Shiga 525-0025 (JP); OKAMOTO, Tomohiro, c/o Ishihara Sangyo Kaisha Ltd., Kusatsu-shi, Shiga 525-0025 (JP); NISHIMURA, Akihiro, c/o Ishihara Sangyo Kaisha Ltd, Kusatsu-shi, Shiga 525-0025 (JP)
(74) Representative: Hartz, Nikolai F., Dr.
(86) International application number: PCT/JP2003/013785
(87) International publication number: WO 2004/039155

(57) **Abstract**

To provide 3-benzoyl-2,4,5-substituted pyridine derivatives or their salts, fungicides containing them, and intermediates for producing them.

By providing a group of compounds comprising specific 3-benzoyl-2,4,5-substituted pyridine derivatives or their salts, or fungicides containing them, which have so-called rainfastness, whereby they are capable of exhibiting excellent effects even when crops have received rainfall after application of the chemicals, it becomes unnecessary to increase the number of applications or the dose of the chemicals by taking into consideration the rainfall after the application of the chemicals. Thus, it is possible to protect crops from disease injuries at a lower cost with less labor.

## Description

### TECHNICAL FIELD

The present invention relates to specific 3-benzoyl-2,4,5-substituted pyridine derivatives or salts thereof, fungicides containing them, and intermediates for producing them.

### BACKGROUND ART

International Publication W002/2527 discloses benzoyl pyridine derivatives useful as fungicides. However, there is no specific disclosure of the compounds of the present invention.

### DISCLOSURE OF THE INVENTION

The benzoyl pyridine derivatives disclosed in WO02/2527 exhibit excellent preventive and curative effects against various diseases, particularly powdery mildew, blast disease, rust, downy mildew, late blight, etc. on rice, cereal, vegetables, fruits and flowering plants. However, there still remains a room for further improvements.

The present inventors have conducted an extensive study relating to benzoyl pyridine derivatives and as a result, have found that specific 3-benzoyl-2,4,5-substituted pyridine derivatives are a group of compounds having so-called rainfastness, which are capable of exhibiting excellent effects even in the case where crops have received rainfall after application of the chemicals. The group of compounds of the present invention have excellent rainfastness, whereby it is unnecessary to increase the number of applications or the dose of the chemicals by taking into consideration the rainfall after application of the chemicals, and it is possible to protect the crops from diseases at a lower cost with less labor.

Namely, the present invention provides a fungicide having rainfastness, which contains at least one 3-benzoyl-2,4,5-substituted pyridine derivative selected from the group consisting of 3-(2,3,4-trimethoxy-6-methylbenzoyl)-4-bromo-5-chloro-2-methoxypyridine, 3-(2,3,4-trimethoxy-6-methylbenzoyl)- 5-chloro-4-ethyl-2-methoxypyridine, 3-(4,5-dimethoxy-2-methylbenzoyl)-4,5-dichloro-2-methoxypyridine, 3-(5-ethoxy-4-methoxy-2-methylbenzoyl)-4,5-dichloro-2-methoxypyridine, 3-(2,3,4-trimethoxy-6-methylbenzoyl)-4-bromo-5-chloro-2-ethoxypyridine, 3-(2,3,4-trimethoxy-6-methylbenzoyl)-5-chloro-2-ethoxy-4-methylpyridine, 3-(2,3,4-trimethoxy-6-methylbenzoyl)-5-bromo-4-chloro-2-ethoxypyridine, 3-(2,3,4-trimethoxy-6-methylbenzoyl)-4-chloro-5-iodo-2-methoxypyridine, 3-(2,3,4-trimethoxy-6-methylbenzoyl)-5-iodo-2,4-dimethoxypyridine, 3-(2,3,4-trimethoxy-6-methylbenzoyl)-5-chloro-2-methoxy-4-methylthiopyridine, 3-(2,3,4-trimethoxy-6-methylbenzoyl)-5-chloro-2,4-dimethoxypyridine, 3-(2,3,4-trimethoxy-6-methylbenzoyl)-4,5-dibromo-2-methoxypyridine, 3-(2,3,4-trimethoxy-6-methylbenzoyl)-4-bromo-2-methoxy-5-methylpyridine, 3-(2,3,4-trimethoxy-6-methylbenzoyl)-5-bromo-4-trifluoromethyl-2-methoxypyridine, 3-(2,3,4-trimethoxy-6-methylbenzoyl)-4,5-dichloro-2-methoxypyridine, 3-(2,3,4-trimethoxy-6-methylbenzoyl)-2,4-dichloro-5-methylpyridine, 3-(2,3,4-trimethoxy-6-methylbenzoyl)-2,4-dichloro-5-iodopyridine, 3-(2,3,4-trimethoxy-6-methylbenzoyl)-2-fluoro-4-iodo-5-methylpyridine, 3-(2,3,4-trimethoxy-6-methylbenzoyl)-2-fluoro-4,5-dimethylpyridine, 3-(2,3,4-trimethoxy-6-methylbenzoyl)-2-methoxy-4,5-dimethylpyridine, 3-(2-ethoxy-3,4-dimethoxy-6-methylbenzoyl)-2-ethoxy-4,5-dimethylpyridine, 3-(2,3,4-trimethoxy-6-methylbenzoyl)-4,5-dimethyl-2-methylthiopyridine, 3-(2,3,4-trimethoxy-6-methylbenzoyl)-5-bromo-4-chloro-2-methoxypyridine, 3-(2,3,4-trimethoxy-6-methylbenzoyl)-4-chloro-2-methoxy-5-methylpyridine, 3-(2,3,4-trimethoxy-6-methylbenzoyl)-2-chloro-5-trifluoromethyl-4-methylpyridine, 3-(2,3,4-trimethoxy-6-methylbenzoyl)-5-trifluoromethyl-2-methoxy-4-methylpyridine, 3-(2,3,4-trimethoxy-6-methylbenzoyl)-2,4-dichloro-5-trifluoromethylpyridine, 3-(2,3,4-trimethoxy-6-methylbenzoyl)-4-chloro-5-trifluoromethyl-2-methoxypyridine, 3-(2,3,4-trimethoxy-6-methylbenzoyl)-5-chloro-4-ethynyl-2-methoxypyridine, 3-(2,3,4-trimethoxy-6-methylbenzoyl)-5-chloro-4-fluoromethyl-2-methoxypyridine, 3-(2,3,4-trimethoxy-6-methylbenzoyl)-5-bromo-4-fluoromethyl-2-methoxypyridine, 3-(2,3,4-trimethoxy-6-methylbenzoyl)-4-fluoromethyl-2-methoxy-5-methylpyridine, 3-(2,3,4-trimethoxy-6-methylbenzoyl)-5-chloro-4-difluoromethyl-2-methoxypyridine, 3-(2,3,4-trimethoxy-6-methylbenzoyl)-5-ethyl-4-trifluoromethyl-2-methoxypyridine, 3-(2,3,4-trimethoxy-6-methylbenzoyl)-5-chloro-2-methoxy-4-methylpyridine, 3-(2,3,4-trimethoxy-6-methylbenzoyl)-5-bromo-2-methoxy-4-methylpyridine, 3-(2,3,4-trimethoxy-6-methylbenzoyl)-4-trifluoromethyl-2-methoxy-5-methylpyridine and 3-(4,5-dimethoxy-2-methylbenzoyl)-5-chloro-2-methoxy-4-methylpyridine, or a salt thereof, as an active ingredient.

The salt of the above 3-benzoyl-2,4,5-substituted pyridine derivative may, for example, be a salt of an inorganic acid such as a hydrochloride, a hydrobromide, a phosphate, a sulfate or a nitrate; or a salt of an organic acid such as an acetate, a benzoate, a p-toluenesulfonate, a methanesulfonate or a propanesulfonate.

The above compounds may be obtained by the production process disclosed in WO02/2527. Otherwise, they may be prepared by using methods in accordance with Journal of Organic Chemistry, 58, 7832 (1993), European Journal of Organic Chemistry, 7, 1371-1376 (2001) and Preparation Examples given hereinafter.

Specifically, they may be produced, for example, by oxidizing at least one phenylpyridylmethanol derivative selected from the group consisting of (2,3,4-trimethoxy-6-methylphenyl)(4-bromo-5-chloro-2-methoxy-3-pyridyl)methanol, (2,3,4-trimethoxy-6-methylphenyl)(5-chloro-4-ethyl-2-methoxy-3-pyridyl)methanol, (4,5-dimethoxy-2-methylphenyl)(4,5-dichloro-2-methoxy-3-pyridyl)methanol, (5-ethoxy-4-methoxy-2-methylphenyl)(4,5-dichloro-2-methoxy-3-pyridyl)methanol, (2,3,4-trimethoxy-6-methylphenyl)(4-bromo-5-chloro-2-ethoxy-3-pyridyl)methanol, (2,3,4-trimethoxy-6-methylphenyl)(5-chloro-2-ethoxy-4-methyl-3-pyridyl)methanol, (2,3,4-trimethoxy-6-methylphenyl)(5-bromo-4-chloro-2-ethoxy-3-pyridyl)methanol, (2,3,4-trimethoxy-6-methylphenyl)(4-chloro-5-iodo-2-methoxy-3-pyridyl)methanol, (2,3,4-trimethoxy-6-methylphenyl)(5-iodo-2,4-dimethoxy-3-pyridyl)methanol, (2,3,4-trimethoxy-6-methylphenyl)(5-chloro-2-methoxy-4-methylthio-3-pyridyl)methanol, (2,3,4-trimethoxy-6-methylphenyl)(5-chloro-2,4-dimethoxy-3-pyridyl)methanol, (2,3,4-trimethoxy-6-methylphenyl)(4,5-dibromo-2-methoxy-3-pyridyl)methanol, (2,3,4-trimethoxy-6-methylphenyl)(4-bromo-2-methoxy-5-methyl-3-pyridyl)methanol, (2,3,4-trimethoxy-6-methylphenyl)(5-bromo-4-trifluoromethyl-2-methoxy-3-pyridyl)methanol, (2,3,4-trimethoxy-6-methylphenyl)(4,5-dichloro-2-methoxy-3-pyridyl)methanol, (2,3,4-trimethoxy-6-methylphenyl)(2,4-dichloro-5-methyl-3-pyridyl)methanol, (2,3,4-trimethoxy-6-methylphenyl)(2,4-dichloro-5-iodo-3-pyridyl)methanol, (2,3,4-trimethoxy-6-methylphenyl)(2-fluoro-4-iodo-5-methyl-3-pyridyl)methanol, (2,3,4-trimethoxy-6-methylphenyl)(2-fluoro-4,5-dimethyl-3-pyridyl)methanol, (2,3,4-trimethoxy-6-methylphenyl)(2-methoxy-4,5-dimethyl-3-pyridyl)methanol, (2-ethoxy-3,4-dimethoxy-6-methylphenyl)(2-ethoxy-4,5-dimethyl-3-pyridyl)methanol, (2,3,4-trimethoxy-6-methylphenyl)(4,5-dimethyl-2-methylthio-3-pyridyl)methanol, (2,3,4-trimethoxy-6-methylphenyl)(5-bromo-4-chloro-2-methoxy-3-pyridyl)methanol, (2,3,4-trimethoxy-6-methylphenyl)(4-chloro-2-methoxy-5-methyl-3-pyridyl)methanol, (2,3,4-trimethoxy-6-methylphenyl)(2-chloro-5-trifluoromethyl-4-methyl-3-pyridyl)methanol, (2,3,4-trimethoxy-6-methylphenyl)(5-trifluoromethyl-2-methoxy-4-methyl-3-pyridyl)methanol, (2,3,4-trimethoxy-6-methylphenyl)(2,4-dichloro-5-trifluoromethyl-3-pyridyl)methanol, (2,3,4-trimethoxy-6-methylphenyl)(4-chloro-5-trifluoromethyl-2-methoxy-3-pyridyl)methanol, (2,3,4-trimethoxy-6-methylphenyl)(5-chloro-4-ethynyl-2-methoxy-3-pyridyl)methanol, (2,3,4-trimethoxy-6-methylphenyl)(5-chloro-4-fluoromethyl-2-methoxy-3-pyridyl)methanol, (2,3,4-trimethoxy-6-methylphenyl)(5-bromo-4-fluoromethyl-2-methoxy-3-pyridyl)methanol, (2,3,4,-trimethoxy-6-methylphenyl)(4-fluoromethyl-2-methoxy-5-methyl-3-pyridyl)methanol, (2,3,4-trimethoxy-6-methylphenyl)(5-chloro-4-difluoromethyl-2-methoxy-3-pyridyl)methanol, (2,3,4-trimethoxy-6-methylphenyl)(5-ethyl-4-trifluoromethyl-2-methoxy-3-pyridyl)methanol, (2,3,4-trimethoxy-6-methylphenyl)(5-chloro-2-methoxy-4-methyl-3-pyridyl)methanol, (2,3,4-trimethoxy-6-methylphenyl)(5-bromo-2-methoxy-4-methyl-3-pyridyl)methanol, (2,3,4-trimethoxy-6-methylphenyl)(4-trifluoromethyl-2-methoxy-5-methyl-3-pyridyl)methanol and (4,5-dimethoxy-6-methylphenyl)(5-chloro-2-methoxy-4-methyl-3-pyridyl)methanol, or a salt thereof.

Further, as the 3-benzoyl-2,4,5-substituted pyridine derivative or a salt thereof, as an active ingredient of a fungicide having rainfastness, it is possible to use, in addition to those mentioned above, at least one 3-benzoyl-2,4,5-substituted pyridine derivative selected from the group consisting of 3-(4,5-dimethoxy-2-methylbenzoyl)-4-bromo-5-chloro-2-methoxypyridine, 3-(4,5-dimethoxy-2-methylbenzoyl)-5-chloro-4-ethyl-2-methoxypyridine, 3-(4,5-dimethoxy-2-methylbenzoyl)-4-bromo-5-chloro-2-ethoxypyridine, 3-(4,5-dimethoxy-2-methylbenzoyl)-5-chloro-2-ethoxy-4-methylpyridine, 3-(4,5-dimethoxy-2-methylbenzoyl)-5-bromo-4-chloro-2-ethoxypyridine, 3-(4,5-dimethoxy-2-methylbenzoyl)-4-chloro-5-iodo-2-methoxypyridine, 3-(4,5-dimethoxy-2-methylbenzoyl)-5-iodo-2,4-dimethoxypyridine), 3-(4,5-dimethoxy-2-methylbenzoyl)-5-chloro-2-methoxy-4-methylthiopyridine, 3-(4,5-dimethoxy-2-methylbenzoyl)-5-chloro-2,4-dimethoxypyridine, 3-(4,5-dimethoxy-2-methylbenzoyl)-4,5-dibromo-2-methoxypyridine, 3-(4,5-dimethoxy-2-methylbenzoyl)-4-bromo-2-methoxy-5-methylpyridine, 3-(4,5-dimethoxy-2-methylbenzoyl)-5-bromo-4-trifluoromethyl-2-methoxypyridine, 3-(4,5-dimethoxy-2-methylbenzoyl)-4,5-dichloro-2-methoxypyridine, 3-(4,5-dimethoxy-2-methylbenzoyl)-2,4-dichloro-5-methylpyridine, 3-(4,5-dimethoxy-2-methylbenzoyl)-2,4-dichloro-5-iodopyridine, 3-(4,5-dimethoxy-2-methylbenzoyl)-2-fluoro-4-iodo-5-methylpyridine, 3-(4,5-dimethoxy-2-methylbenzoyl)-2-fluoro-4,5-dimethylpyridine, 3-(4,5-dimethoxy-2-methylbenzoyl)-2-ethoxy-4,5-dimethylpyridine, 3-(4,5-dimethoxy-2-methylbenzoyl)-4,5-dimethyl-2-methylthiopyridine, 3-(4,5-dimethoxy-2-methylbenzoyl)-5-bromo-4-chloro-2-methoxypyridine, 3-(4,5-dimethoxy-2-methylbenzoyl)-4-chloro-2-methoxy-5-methylpyridine, 3-(4,5-dimethoxy-2-methylbenzoyl)-2-chloro-5-trifluoromethyl-4-methylpyridine, 3-(4,5-dimethoxy-2-methylbenzoyl)-5-trifluoromethyl-2-methoxy-4-methylpyridine, 3-(4,5-dimethoxy-2-methylbenzoyl)-2,4-dichloro-5-trifluoromethylpyridine, 3-(4,5-dimethoxy-2-methylbenzoyl)-4-chloro-5-trifluoromethyl-2-methoxypyridine, 3-(4,5-dimethoxy-2-methylbenzoyl)-5-chloro-4-ethynyl-2-methoxypyridine, 3-(4,5-dimethoxy-2-methylbenzoyl)-5-chloro-4-fluoromethyl-2-methoxypyridine, 3-(4,5-dimethoxy-2-methylbenzoyl)-5-bromo-4-fluoromethyl-2-methoxypyridine, 3-(4,5-dimethoxy-2-methylbenzoyl)-4-fluoromethyl-2-methoxy-5-methylpyridine, 3-(4,5-dimethoxy-2-methylbenzoyl)-5-chloro-4-difluoromethyl-2-methoxypyridine, 3-(4,5-dimethoxy-2-methylbenzoyl)-5-bromo-2-methoxy-4-methylpyridine and 3-(4,5-dimethoxy-2-methylbenzoyl)-4-trifluoromethyl-2-methoxy-5-methylpyridine, or a salt thereof.

### BEST MODE FOR CARRYING OUT THE INVENTION

The above 3-benzoyl-2,4,5-substituted pyridine derivative or its salt is useful as an active ingredient for a fungicide, particularly as an active ingredient for an agricultural and horticultural fungicide. As the agricultural and horticultural fungicide, it is effective for controlling diseases such as blast, brown spot or sheath blight of rice (Oryza sativa); powdery mildew, scab, rust, snow mold, loose smut, eyespot, leaf spot or glume blotch of cereal (Tricum sativum, Hordeum vulgare, Avena sativa, etc.); melanose or scab of citrus (Citrus spp.); blossom blight, powdery mildew, Altenaria leaf spot or scab of apple (Malus pumila); scab or black spot of pear (Pyrus spp.); brown rot, scab or Phomopsis rot of peach (Prunus spp.); Anthracnose, ripe rot, powdery mildew or downy mildew of grape (Vitis spp.); anthracnose or circular leaf spot of Japanese persimmon (Diospyros spp.); anthracnose, powdery mildew, gummy stem blight or downy mildew of cucurbit (Cucumis spp.); early blight, leaf mold or late blight of tomato (Lycopersicon esculentum); Alternaria leaf spot of crucifer (Brassica sp., Raphanus sp., etc); early blight or late blight of potato (Solanum tuberosum); powdery mildew of strawberry (Fragaria X ananasa); gray mold or stem rot of various crops. It shows an excellent controlling effect particularly on powdery mildew of cereal and vegetables and rice blast. Further, it is also effective for controlling soil-borne diseases caused by phytopathogenic fungi such as Fusarium, Pythium, Rhizoctonia, Verticillium and Plasmodiophora.

Further, the active ingredient compound of the present invention (hereinafter referred to simply as the compound of the present invention) has excellent rainfastness and maintains the above controlling effects even when the crops have received rainfall after the application of the chemical. Thus, it is effective for use under frequent rainfall conditions.

The compound of the present invention may be used usually in combination with an agricultural adjuvant to formulate various preparations, such as a dust, granules, a granular wettable powder, a wettable powder, an aqueous suspension, an oil suspension, a water soluble powder, an emulsifiable concentrate, an aqueous solution, a paste, an aerosol or a microdose dusting powder. The compound of the present invention may be formed into any preparation which is usually used in the agricultural and horticultural field so long as the purpose of the present invention is met. The adjuvant to be used for preparation may, for example, be a solid carrier such as diatomaceous earth, hydrated lime, calcium carbonate, talc, white carbon, kaolin, bentonite, a mixture of kaolinite and sericite, clay, sodium carbonate, sodium bicarbonate, glauber's salt, zeolite or starch; a solvent such as water, toluene, xylene, solvent naphtha, dioxane, acetone, isophorone, methyl isobutyl ketone, chlorobenzene, cyclohexane, dimethylsulfoxide, dimethylformamide, dimethylacetamide, N-methyl-2-pyrrolidone or an alcohol; an anionic surfactant or spreading agent such as a fatty acid salt, a benzoate, an alkyl sulfosuccinate, a dialkyl sulfosuccinate, a polycarboxylate, an alkyl sulfuric ester salt, an alkyl sulfate, an alkyl aryl sulfate, an alkyl diglycol ether sulfate, an alcohol sulfuric ester salt, an alkyl sulfonate, an alkyl aryl sulfonate, an aryl sulfonate, a lignin sulfonate, an alkyl diphenyl ether disulfonate, a polystyrene sulfonate, an alkyl phosphoric ester salt, an alkyl aryl phosphate, a styryl aryl phosphate, a polyoxyethylene alkyl ether sulfuric ester salt, a polyoxyethylene alkyl aryl ether sulfate, a polyoxyethylene alkyl aryl ether sulfuric ester salt, a polyoxyethylene alkyl ether phosphate, a polyoxyethylene alkyl aryl phosphoric ester salt or a salt of a naphthalene sulfonic acid formalin condensate; a nonionic surfactant or spreading agent such as a sorbitan fatty acid ester, a glycerol fatty acid ester, a fatty acid polyglyceride, a fatty acid alcohol polyglycol ether, an acetylene glycol, an acetylene alcohol, an oxyalkylene block polymer, a polyoxyethylene alkyl ether, a polyoxyethylene alkyl aryl ether, a polyoxyethylene styryl aryl ether, a polyoxyethylene glycol alkyl ether, a polyoxyethylene fatty acid ester, a polyoxyethylene sorbitan fatty acid ester, a polyoxyethylene glycerol fatty acid ester, a polyoxyethylene hardened caster oil or a polyoxypropylene fatty acid ester; vegetable oil or mineral oil such as olive oil, kapok oil, caster oil, palm oil, camellia oil, coconut oil, sesame oil, corn oil, rice bran oil, peanut oil, cotton oil, soy bean oil, rape oil, linseed oil, tung oil or liquid paraffin. Such an adjuvant may be selected for use from adjuvants which are known in the agricultural and horticultural field within a range of not departing from the object of the present invention. Further, an adjuvant which is usually used may also be employed, such as a bulking agent, a thickener, an anti-settling agent, a freeze proofing agent, a dispersion stabilizer, a crop injury-reducing agent or a mildewproofing agent. The blending proportion of the compound of the present invention to the adjuvant is generally from 0.005:99.995 to 95:5, preferably from 0.2:99.8 to 90:10. These formulations can be practically used either as they are or after they are diluted with a diluent such as water to predetermined concentrations, and a spreading agent may be added thereto as the case may be required.

The concentration of the compound of the present invention varies depending upon the crop plant as the object, the way of application, the form of preparation or the dose, and hence cannot be generically determined. However, in the case of foliage treatment, the concentration of the compound as the active ingredient is generally from 0.1 to 10,000 ppm, preferably from 1 to 2,000 ppm. In the case of soil treatment, it is generally from 10 to 100,000 g/ha, preferably from 200 to 20,000 g/ha.

The compound of the present invention in the form of a various formulation or a diluted product thereof can be applied by an application method which is commonly used, such as spreading (spreading, spraying, misting, atomizing, grain diffusing or application on water), soil application (such as mixing or irrigation) or surface application (such as coating, dust coating or covering). Further, it may be applied also by so-called ultra low volume. By this method, the preparation can contain 100% of the active ingredient.

The compound of the present invention may be mixed or used together with e.g. another agricultural chemical such as a fungicide, an insecticide, a miticide, a nematicide, an antiviral agent, an attractant, an herbicide or a plant growth regulator. In such a case, a still more excellent effect may be obtained sometimes.

Among such other agricultural chemicals, examples of the active ingredient compounds of the fungicides (generic names; including compounds which are under application) include pyrimidinamine type compounds such as Mepanipyrim, Pyrimethanil and Cyprodinil; pyridinamine type compounds such as Fluazinam;
azole type compounds such as Triadimefon, Bitertanol, Triflumizole, Etaconazole, Propiconazole, Penconazole, Flusilazole, Myclobutanil, Cyproconazole, Terbuconazole, Hexaconazole, Furconazole-cis, Prochloraz, Metconazole, Epoxiconazole, Tetraconazole, Oxpoconazole fumarate, Sipconazole and Prothioconazole;
quinoxaline type compounds such as Quinomethionate;
dithiocarbamate type compounds such as Maneb, Zineb, Mancozeb, Polycarbamate, Metiram and Propineb;
organic chlorine type compounds such as Fthalide, Chlorothalonil and Quintozene;
imidazole type compounds such as Benomyl, Thiophanate-Methyl, Carbendazim and Cyazofamid;
cyanoacetamide type compounds such as Cymoxanil;
phenylamide type compounds such as Metalaxyl, Metalaxyl M, Oxadixyl, Ofurace, Benalaxyl, Furalaxyl and Cyprofuram;
sulfenic acid type compounds such as Dichlofluanid;
copper type compounds such as Cupric hydroxide and Oxine Copper;
isoxazole type compounds such as Hymexazol;
organophosphorus compounds such as Fosetyl-Al, Tolcofos-Methyl, S-benzyl O, O-diisopropylphosphorothioate, O-ethyl S,S-diphenylphosphorodithioate and aluminum ethyl hydrogen phosphonate;
N-halogenothioalkyl type compounds such as Captan, Captafol and Folpet;
dicarboxyimide type compounds such as Procymidone, Iprodione and Vinclozolin;
benzanilide type compounds such as Flutolanil, Mepronil, Zoxamid and Tiadinil;
piperazine type compounds such as Triforine;
pyridine type compounds such as Pyrifenox;
carbinol type compounds such as Fenarimol and Flutriafol;
piperidine type compounds such as Fenpropidine;
morpholine type compounds such as Fenpropimorph; organotin type compounds such as Fentin Hydroxide and Fentin Acetate;
urea type compounds such as Pencycuron;
cinnamic acid type compounds such as Dimethomorph and Flumorph;
phenyl carbamate type compounds such as Diethofencarb;
cyanopyrrole type compounds such as Fludioxonil and Fenpiclonil;
strobilurin type compounds such as Azoxystrobin, Kresoxim-Methyl, Metominofen, Triflouxystrobin, Picoxystrobin, Oryzastrobin, Dimoxystrobin and Fluoxastrobin;
oxazolidinone type compounds such as Famoxadone;
thiazole carboxamide type compounds such as Ethaboxam;
silyl amide type compounds such as Silthiopham;
aminoacid amidecarbamate type compounds such as Iprovalicarb and Benthiavalicarb;
Imidazolidine type compounds such as Fenamidone;
hydroxyanilide type compounds such as Fenhexamid;
benzene sulfonamide type compounds such as Flusulfamid;
oxime ether type compounds such as Cyflufenamid;
phenoxyamide type compounds such as Fenoxanil;
triazole type compounds such as Simeconazole;
anthraquinone type compounds; crotonic acid type compounds; antibiotics and other compounds such as Isoprothiolane, Tricyclazole, Pyroquilon, Diclomezine, Probenazole, Quinoxyfen, Propamocarb Hydrochloride, Spiroxamine, Chloropicrin, Dazomet, Metam-sodium, Nicobifen, Metrafenone, MTF-753, UBF-307, Diclocymet and Proquinazid.

Among above other agricultural chemicals, examples of the active ingredient compounds (generic names; including compounds which are under application) of the insecticide, miticide or nematicide i.e. a pesticide include organic phosphate type compounds such as Profenofos, Dichlorvos, Fenamiphos, Fenitrothion, EPN, Diazinon, Chlorpyrifos-methyl, Acephate, Prothiofos, Fosthiazate, Phosphocarb, Cadusafos and Dislufoton;
carbamate type compounds such as Carbaryl, Propoxur, Aldicarb, Carbofuran, Thiodicarb, Methomyl, Oxamyl, Ethiofencarb, Pirimicarb, Fenobucarb, Carbosulfan and Benfuracarb;
nelicetoxin derivatives such as Cartap and Thiocyclam;
organic chlorine type compounds such as Dicofol and Tetradifon;
organic metal type compounds such as Fenbutatin Oxide;
pyrethroid type compounds such as Fenvalerate, Permethrin, Cypermethrin, Deltamethrin, Cyhalothrin, Tefluthrin, Ethofenprox, Flufenprox and Imidate;
benzoyl urea type compounds such as Diflubenzuron, Chlorfluazuron, Teflubenzuron, Flufenoxuron, Bistrifluron and Noviflumuron;
juvenile hormone-like compounds such as Methoprene;
pyridazinone type compounds such as Pyridaben;
pyrazole type compounds such as Fenpyroximate, Fipronil, Tebufenpyrad, Ethiprole, Tolefenpyrad and Acetoprole;
neonicotinoides such as Imidacloprid, Nitenpyram, Acetamiprid, Thiacloprid, Thiamethoxam, Clothianidin, Nidinotefuran and Dinotefuran;
hydrazine type compounds such as Tebufenozide, Methoxyfenozide and Chromafenozide;
pyridine type compounds such as Pyridaryl and Flonicamid;
tetronic acid type compounds such as Spirodiclofen;
strobilurin type compounds such as Fluacrypyrin;
pyridinamine type compounds such as Flufenerium;
dinitro type compounds, organosulfur compounds, urea type compounds, triazine type compounds, hydrozone type compounds and other compounds such as Buprofezin, Hexythiazox, Amitraz, Chlordimeform, Silafluofen, Triazamate, Pymetrozine, Pyrimidifen, Chlorfenapyr, Indoxacarb, Acequinocyl, Etoxazole, Cyromazine, 1,3-dichloropropene, Verbutin, Spiromesifen, Thiazolylcinnanonitrile and Amidoflumet; AKD-1022 and IKA-2000. Further, the fungicide of the present invention may also be mixed or used together with a microbial pesticide such as a BT agent or an insect pathogenic virus agent or an antibiotic such as Avermectin, Milbemycin, Spinosad or Emamectin Benzoate.

### EXAMPLES

Now, specific Synthesis Examples of the benzoylpyridine derivative of the present invention and the intermediate for its production will be described below.

### SYNTHESIS EXAMPLE 1

### Synthesis of 3-(2,3,4-trimethoxy-6-methylbenzoyl)-4,5-dichloro-2-methoxypyridine (compound No. 1-15)

(a) 222 mL of n-butyllithium (1.57 mol/L hexane solution) was dropwise added at -20°C to a solution having 34.2 g (340 mmol) of diisopropylamine dissolved in 400 mL of tetrahydrofuran, followed by stirring for 1 hour. The solution was cooled to -78°C, a solution having 32.0 g (330 mmol) of 2-fluoropyridine dissolved in 50 mL of tetrahydrofuran was added thereto, followed by stirring for 4 hours, and a solution having 87.1 g (341 mmol) of iodine dissolved in 150 mL of tetrahydrofuran was added thereto, followed by stirring for 1 hour. 200 mL of water was added to the mixture to terminate the reaction, and tetrahydrofuran was distilled off under reduced pressure. Extraction with ethyl ether was carried out, the organic layer was dried over anhydrous sodium sulfate and subjected to filtration, and the solvent was distilled off under reduced pressure, to obtain 67.4 g (crude yield 92%) of a crude product of 2-fluoro-3-iodopyridine.
   ¹H-NMR(CDCl₃, 400 MHz) : δ (ppm) = 6.91-6.88(m,1H), 8.08-8.12(m,2H)
(b) 189 mL of n-butyllithium (1.57 mol/L hexane solution) was dropwise added at -20°C to a solution having 30.2 g (302 mmol) of diisopropylamine dissolved in 380 mL of tetrahydrofuran, followed by stirring for 1 hour. The solution was cooled to -78°C, a solution having 67.4 g (302 mmol) of the crude product of 2-fluoro-3-iodopyridine obtained in step (a) dissolved in 100 mL of tetrahydrofuran was added thereto, followed by stirring for 1 hour. 300 mL of water was added to the mixture to terminate the reaction, and tetrahydrofuran was distilled off under reduced pressure. Extraction with ethyl ether was carried out, the organic layer was dried over anhydrous sodium sulfate and subjected to filtration, and the solvent was distilled off under reduced pressure, to obtain 59.3 g (crude yield 89%) of a crude product of 2-fluoro-4-iodopyridine.
   ¹H-NMR(CDCl₃, 400 MHz) : δ (ppm) = 7.33(d,1H, J = 2.8 Hz), 7.51(d,1H, J = 5.2 Hz), 7.88(dd,1H, J=5.2 Hz, 2.8 Hz)
(c) 59.4 g (253 mmol) of the crude product of 2-fluoro-4-iodopyridine obtained in step (b) was added to 500 mL of methanol, and 21.5 g (398 mmol) of sodium methoxide was added, followed by refluxing by heating for 3 hours. 300 mL of water was added to terminate the reaction, and methanol was distilled off under reduced pressure. Extraction with ethyl ether was carried out, then, the organic layer was dried over sodium sulfate and subjected to filtration, and the solvent was distilled off under reduced pressure to obtain 56.7 g (crude yield 91%) of a crude product of 4-iodo-2-methoxypyridine.
   ¹H-NMR(CDCl₃, 400 MHz) : δ (ppm) = 3.86(s,3H), 7.12-7.16(m,2H), 7.79(d,1H, J = 5.6 Hz)
(d) 50.6 mL of isopropyl magnesium chloride (2 mol/L tetrahydrofuran solution) was cooled with ice, and a solution having 19.8 g (84.3 mmol) of the crude product of 4-iodo-2-methoxypyridine obtained in step (c) dissolved in 80 mL of tetrahydrofuran, was added, followed by stirring at 0°C for 1 hour and at room temperature for 1 hour. Then, 16.9 g (127 mmol) of N-chlorosuccinimide was gradually added, followed by stirring at room temperature for 1 hour. 100 mL of water was added to terminate the reaction, and tetrahydrofuran was distilled off under reduced pressure. Extraction with ethyl ether was carried out, then the organic layer was dried over sodium sulfate and subjected to filtration, and the solvent was distilled off under reduced pressure to obtain 11.0 g (crude yield 91%) of a crude product of 4-chloro-2-methoxypyridine.
   ¹H-NMR(CDCl₃, 400 MHz) : δ (ppm) = 3.91(s, 3H), 6.70(d, 1H, J = 2.0 Hz), 6.81(dd, 1H, J = 6.0 Hz, 2.0 Hz), 7.99(d,1H, J = 6.0 Hz)
(e) 10.0 g (69.9 mmol) of the crude product of 4-chloro-2-methoxypyridine obtained in step (d) was dissolved in 100 mL of dimethylformamide, and 37.2 g (279 mmol) of N-chlorosuccinimide was added, followed by stirring at room temperature for 12 hours. 400 mL of water was added to terminate the reaction. Extraction with ethyl ether was carried out, then the organic layer was washed with a saturated sodium chloride aqueous solution, dried over sodium sulfate and subjected to filtration, and the solvent was distilled off under reduced pressure to obtain 9.10 g (crude yield 73%) of a crude product of 4,5-dichloro-2-methoxypyridine.
   ¹H-NMR(CDCl₃, 400 MHz) : δ (ppm) = 3.90(s, 3H), 6.85(s, 1H), 8.14(s, 1H)
(f) 15.1 mL of n-butyllithium (1.57 mol/L hexane solution) was dropwise added at -20°C to a solution having 2.40 g (23.7 mmol) of diisopropylamine dissolved in 30 mL of tetrahydrofuran, followed by stirring for 1 hour. The solution was cooled to -78°C, a solution having 4.22 g (23.6 mmol) of 4,5-dichloro-2-methoxypyridine obtained in step (e) dissolved in 20 mL of tetrahydrofuran was added, followed by stirring for 2 hours, and then a solution having 5.00 g (23.8 mmol) of 2,3,4-trimethoxy-6-methylbenzaldehyde dissolved in 20 mL of tetrahydrofuran was added, followed by stirring for 30 minutes. 50 mL of water was added to the mixture to terminate the reaction, and tetrahydrofuran was distilled off under reduced pressure. Extraction with ethyl ether was carried out, then the organic layer was .dried over sodium sulfate and subjected to filtration, and the solvent was distilled off under reduced pressure, followed by purification by silica gel column chromatography, to obtain 4.66 g (crude yield 51%) of (2,3,4-trimethoxy-6-methylphenyl)(4,5-dichloro-2-methoxy-3-pyridyl)methanol.
   ¹H-NMR(CDCl₃, 400 MHz) : δ (ppm) = 2.32(s, 3H), 3.52(s, 3H), 3.77(s, 3H), 3.82(s, 3H), 4.11(s, 3H), 5.32(d, 1H, J=10.0 Hz), 6.21(d, 1H, J=10.0 Hz), 6,55(s, 1H), 8.07(s, 1H)
(g) 13.8 g (159 mmol) of manganese dioxide was added to a solution having 4.66 g (12.0 mmol) of (2,3,4-trimethoxy-6-methylphenyl)(4,5-dichloro-2-methoxy-3-pyridyl)methanol obtained in step (f) dissolved in 30 mL of toluene, followed by refluxing by heating for 2 hours. After cooling to room temperature, manganese dioxide was removed by filtration on the pad of celite, and then, toluene was distilled off under reduced pressure, followed by purification by silica gel column chromatography to obtain 2.98 g (yield 65%) of 3-(2,3,4-trimethoxy-6-methylbenzoyl)-4,5-dichloro-2-methoxypyridine.
   ¹H-NMR(CDCl₃, 400 MHz) : δ (ppm) = 2.46(s,3H), 3.45(s,3H), 3.74(s,3H), 3.90(s,3H), 4.00(s,3H), 6.55(s,1H), 8.13(s,1H)

### SYNTHESIS EXAMPLE 2

### Synthesis of 3-(2,3,4-trimethoxy-6-methylbenzoyl)-2-methoxy-4,5-dimethylpyridine (compound No. 1-20)

(a) 26.5 mL of n-butyllithium (1.57 mol/L hexane solution) was dropwise added at -78°C to a solution having 4.02 g (39.8 mmol) of diisopropylamine dissolved in 70 mL of tetrahydrofuran, followed by stirring for 30 minutes. To this solution, a solution having 4.42 g (39.8 mmol) of 2-fluoro-5-methylpyridine dissolved in 18 mL of tetrahydrofuran was added, followed by stirring for 4 hours. Then, a solution having 10.1 g (39.8 mmol) of iodine dissolved in 27 mL of tetrahydrofuran was added, followed by stirring for 2 hours. 16 mL of water and 120 mL of an aqueous sodium thiosulfate solution were added, and extraction with ethyl ether was carried out. Then, the organic layer was dried over magnesium sulfate and subjected to filtration, and the solvent was distilled off under reduced pressure. The obtained crude product was purified by silica gel column chromatography to obtain 3.15 g (yield: 33%) of 2-fluoro-3-iodo-5-methylpyridine.
   ¹H-NMR(CDCl₃, 400 MHz) : δ (ppm) = 2.27(s, 3H), 7.95(m, 2H)
(b) 8.90 mL of n-butyllithium (1.57 mol/L hexane solution) was dropwise added at -78°C to a solution having 1.34 g (13.3 mmol) of diisopropylamine dissolved in 27 mL of tetrahydrofuran, followed by stirring for 30 minutes. To this solution, a solution having 3.15 g (13.3 mmol) of 2-fluoro-3-iodo-5-methylpyridine obtained in step (a) dissolved in 5 mL of tetrahydrofuran was added, followed by stirring for 1 hour. Then, a solution having 2.79 g (13.3 mmol) of 2,3,4-trimethoxy-6-methylbenzaldehyde dissolved in 5 mL of tetrahydrofuran was added, followed by stirring for 2 hours. After raising the temperature to room temperature, 50 mL of water was added, and extraction with ethyl ether was carried out. Then, the organic layer was dried over magnesium sulfate and subjected to filtration, and the solvent was distilled off under reduced pressure. The obtained crude product was purified by silica gel column chromatography to obtain 4.45 g (yield 75%) of (2,3,4,-trimethoxy-6-methylphenyl)(2-fluoro-4-iodo-5-methyl-3-pyridyl)methanol.
   ¹H-NMR(CDCl₃, 400 MHz) : δ (ppm) = 2.21(s, 3H), 2.42(s, 3H), 3.72(s, 3H), 3.79(s, 3H), 3.81(s, 3H), 4.97(d, 1H, J = 10.0 Hz), 6.08(d, 1H, J = 10.0 Hz), 6.46(s, 1H), 7.86(s, 1H)
(c) 17.3 g (0.18 mol) of manganese dioxide was added to a solution having 4.35 g (9.70 mmol) of (2,3,4-trimethoxy-6-methylphenyl) (2-fluoro-4-iodo-5-methyl-3-pyridyl)methanol obtained in step (b) dissolved in 130 mL of toluene, followed by refluxing by heating for 2 hours. After cooling to room temperature, manganese dioxide was removed by filtration on the pad of celite. Then, toluene was distilled off under reduced pressure, followed by purification by silica gel column chromatography to obtain 2.80 g (yield 65%) of 3-(2,3,4-trimethoxy-6-methylbenzoyl)-2-fluoro-4-iodo-5-methylpyridine (melting point 140-141°C).
   ¹H-NMR(CDCl₃, 400 MHz) : δ (ppm) = 2.41(s, 3H), 2.50(s, 3H), 3.42(s, 3H), 3.90(s, 3H), 3.74(s, 3H), 6.57(s, 1H), 7.94(s, 1H)
(d) 1.50 g (3.37 mmol) of 3-(2,3,4-trimethoxy-6-methylbenzoyl)-2-fluoro-4-iodo-5-methylpyridine obtained in step (c), 1.40 g (10.1 mmol) of potassium carbonate, 0.39 g (0.34 mmol) of tetrakis(triphenylphosphine)palladium, 15 mL of dioxane and 0.42 g (1.67 mmol) of 50% trimethylboroxin were mixed, followed by refluxing by heating for 6 hours. After cooling to room temperature, filtration on the pad of celite was carried out, followed by washing with ethyl acetate and tetrahydrofuran. Then, the solvent was distilled off under reduced pressure, and the obtained crude product was purified by silica gel column chromatography to obtain 0.79 g (yield 70%) of 3-(2,3,4-trimethoxy-6-methylbenzoyl)-2-fluoro-4,5-dimethylpyridine.
   ¹H-NMR(CDCl₃, 400 MHz) : δ (ppm) = 2.28(s, 3H), 2.32(s, 3H), 2.42(s, 3H), 3.35(s, 3H), 3.74(s, 3H), 3.90(s, 3H), 6.57(s, 1H), 7.94(s, 1H)
(e) A solution having 0.06 g (1.5 mmol) of 60% sodium hydride dissolved in 1 mL of methanol was dropwise added to a solution having 0.20 g (0.60 mmol) of 3-(2,3,4-trimethoxy-6-methylbenzoyl)-2-fluoro-4,5-dimethylpyridine obtained in step (d) dissolved in 2.5 mL of methanol, followed by refluxing by heating for 16 hours. After cooling to room temperature, 5 mL of water was added, and the mixture was weakly acidified with dilute hydrochloric acid. Extraction with ethyl ether was carried out, followed by washing with an aqueous sodium chloride solution. The organic layer was dried over magnesium sulfate and subjected to filtration, and the solvent was distilled off under reduced pressure. The obtained crude product was purified by silica gel column chromatography to obtain 89.0 mg (yield 43%) of 3-(2,3,4-trimethoxy-6-methylbenzoyl)-2-methoxy-4,5-dimethylpyridine.
   ¹H-NMR(CDCl₃, 400 MHz) : δ (ppm) = 2.19(s, 3H), 2.21(s, 3H), 2.39(s, 3H), 3.24(s, 3H), 3.70(s, 3H), 3.74(s, 3H), 3.87(s, 3H), 6.53(s, 1H), 7.87(s, 1H)

### SYNTHESIS EXAMPLE 3

### Synthesis of 3-(2,3,4-trimethoxy-6-methylbenzoyl)-5-chloro-2-methoxy-4-methylpyridine (Compound No. 1-35)

2.29 g (16.6 mmol) potassium carbonate and 1.38 g (5.49 mmol) of trimethylboroxin (50 wt% tetrahydrofuran solution) were added to a solution having 2.10 g (5.44 mmol) of 3-(2,3,4-trimethoxy-6-methylbenzoyl)-4,5-dichloro-2-methoxypyridine (Compound No. 1-15) and 0.64 g (0.55 mmol) of tetrakis(triphenylphosphine)palladium dissolved in 30 mL of 1,4-dioxane, followed by stirring under reflux by heating for 10 hours. Water was added to terminate the reaction, and the aqueous layer was extracted with ethyl acetate and then dried over anhydrous sodium sulfate and subjected to filtration. The solvent was distilled off under reduced pressure. The crude product was purified by silica gel column chromatography to obtain 1.49 g (yield 75%) of 3-(2,3,4-trimethoxy-6-methylbenzoyl)-5-chloro-2-methoxy-4-methylpyridine.
¹H-NMR(CDCl₃, 400 MHz) : δ (ppm) = 2.31(s, 3H), 2.40(s, 3H), 3.30(s, 3H), 3.73(s, 3H), 3.74(s, 3H), 3.88(s, 3H), 6.54(s, 1H), 8.06(s, 1H)

### SYNTHESIS EXAMPLE 4

### Synthesis of 3-(2,3,4-trimethoxy-6-methylbenzoyl)-4-trifluoromethyl-2-methoxy-5-methylpyridine (Compound No. 1-37)

(a) A solution having 5.05 g (27.8 mmol) of 2-chloro-4-trifluoromethylpyridine and 3.59 g (66.5 mmol) of sodium methoxide dissolved in 40 mL of methanol was stirred under reflux by heating for 4 hours. Water was added to terminate the reaction, and extraction with diethyl ether was carried out. Then, the organic layer was dried over anhydrous sodium sulfate and subjected to filtration on a silica gel cake. The solvent was distilled off under reduced pressure to obtain 4.19 g (yield 85%) of 4-trifluoromethyl-2-methoxypyridine.
   ¹H-NMR(CDCl₃, 400 MHz) : δ (ppm) = 3.96(s, 3H), 6.95(s, 1H), 7.05(d, 1H, J = 5.2 Hz), 8.29(d, 1H, J = 5.2 Hz)
(b) 4.00 mL (78.1 mmol) of bromine was dropwise added to a solution having 8.21 g (46.4 mmol) of 4-trifluoromethyl-2-methoxypyridine obtained in step (a) and 7.98 g (97.3 mmol) of sodium acetate dissolved in 15 mL of acetic acid, followed by stirring for 4 days. An aqueous potassium hydroxide solution was added to terminate the reaction, and extraction with diethyl ether was carried out. Then, the organic layer was dried over anhydrous sodium sulfate and subjected to filtration on a silica gel cake. The solvent was distilled off under reduced pressure to obtain 5.81 g of a mixture of 5-bromo-4-trifluoromethyl-2-methoxypyridine and the raw material 4-trifluoromethyl-2-methoxypyridine (molar ratio of 55:45).
   ¹H-NMR(CDCl₃, 400 MHz) : δ (ppm) = 3.94(s, 3H), 7.03(s, 1H), 8.37(s, 1H)
(c) 17.1 mL of n-butyllithium (1.57 mol/L hexane solution) was dropwise added at 0°C to a solution having 3.80 mL (27.1 mmol) of diisopropylamine dissolved in 50 mL of tetrahydrofuran, followed by stirring for 30 minutes. The solution was cooled to -78°C, and a solution having 5.81 g of the mixture of 5-bromo-4-trifluoromethyl-2-methoxypyridine and 4-trifluoromethyl-2-methoxypyridine (molar ratio of 55:45) obtained in step
(c) dissolved in 10 mL of tetrahydrofuran was added, followed by stirring for 45 minutes. A solution having 5.51 g (26.2 mmol) of 2,3,4-trimethoxy-6-methylbenzaldehyde dissolved in 15 mL of tetrahydrofuran was added, followed by stirring for 1 hour. Water was added to the mixture to terminate the reaction, and tetrahydrofuran was distilled off under reduced pressure. Extraction with ethyl acetate was carried out. Then, the organic layer was dried over anhydrous sodium sulfate and subjected to filtration, and the solvent was distilled off under reduced pressure. The crude product was purified by silica gel column chromatography to obtain 5.02 g of (2,3,4-trimethoxy-6-methylphenyl)(5-bromo-4-trifluoromethyl-2-methoxy-3-pyridyl)methanol.
   ¹H-NMR(CDCl₃, 400 MHz) : δ (ppm) = 2.35(s, 3H), 3.29(s, 3H), 3.74(s, 3H), 3.82(s, 3H), 3.92(s, 3H), 4.87(d, 1H, J = 10.8 Hz), 6.21(d, 1H, J = 10.8 Hz), 6.51(s, 1H), 8.31(s, 1H)
(d) 20.0 g (230 mmol) of manganese dioxide was added to a solution having 4.80 g (10.3 mmol) of (2,3,4-trimethoxy-6-methylphenyl)(5-bromo-4-trifluoromethyl-2-methoxy-3-pyridyl)methanol obtained in step (c) dissolved in 110 mL of toluene, followed by stirring under reflux by heating for 1 hour. After cooling to room temperature, the mixture was subjected to filtration on the pad of celite, and the solvent was distilled off under reduced pressure. The crude product was purified by silica gel column chromatography to obtain 3.93 g (yield 82%) of 3-(2,3,4-trimethoxy-6-methylbenzoyl)-5-bromo-4-trifluoromethyl-2-methoxypyridine.
   ¹H-NMR(CDCl₃, 400 MHz) : δ (ppm) = 2.57(s, 3H), 3.36(s, 3H), 3.75(s, 3H), 3.86(s, 3H), 3.93(s, 3H), 6.59(s, 1H), 8.38(s, 1H)
(e) 3.80 mL (3.80 mmol) of dimethylzinc (1.0 mol/L hexane solution) was dropwise added at 0°C to a solution having 0.60 g (1.29 mmol) of 3-(2,3,4-trimethoxy-6-methylbenzoyl)-5-bromo-4-trifluoromethyl-2-methoxypyridine obtained in step (d) and 0.10 g (0.09 mmol) of tetrakis(triphenylphosphine)palladium dissolved in 10 mL of tetrahydrofuran, and the temperature was naturally raised, followed by stirring at room temperature for 8 days. Water was added to terminate the reaction, and tetrahydrofuran was distilled off under reduced pressure. Extraction with ethyl acetate was carried out. Then, the organic layer was dried over anhydrous sodium sulfate and subjected to filtration, and the solvent was distilled off under reduced pressure. The crude product was purified by silica gel column chromatography to obtain 0.50 g (yield 96%) of 3-(2,3,4-trimethoxy-6-methylbenzoyl)-4-trifluoromethyl-2-methoxy-5-methylpyridine.
   ¹H-NMR(CDCl₃, 400 MHz) : δ (ppm) = 2.41(s, 3H), 2.56(s, 3H), 3.29(s, 3H), 3.74(s, 3H), 3.83(s, 3H), 3.91(s, 3H), 6.58(s, 1H), 8.05(s, 1H)

Compounds of the present invention which are produced by methods in accordance with the above Synthesis Examples 1 to 4 and Synthesis Examples 1 to 16 in WO02/2527 will be shown in the following Tables 1 and 2.

**Table 1**

| No. | Names of compounds | Physical properties |
|---|---|---|
| 1-1 | 3-(2,3,4-trimethoxy-6-methylbenzoyl)-4-bromo-5-chloro-2-methoxypyridine | m.p. 92-94°C |
| 1-2 | 3-(2,3,4-trimethoxy-6-methylbenzoyl)-5-chloro-4-ethyl-2-methoxypyridine | Yellow oily substance |
| 1-3 | 3-(4,5-dimethoxy-2-methylbenzoyl)-4,5-dichloro-2-methoxypyridine | m.p. 139-141°C |
| 1-4 | 3-(5-ethoxy-4-methoxy-2-methylbenzoyl)-4,5-dichloro-2-methoxypyridine | m.p. 110-112°C |
| 1-5 | 3-(2,3,4-trimethoxy-6-methylbenzoyl)-4-bromo-5-chloro-2-ethoxypyridine | Yellow oily substance |
| 1-6 | 3-(2,3,4-trimethoxy-6-methylbenzoyl)-5-chloro-2-ethoxy-4-methylpyridine | Yellow oily substance |
| 1-7 | 3-(2,3,4-trimethoxy-6-methylbenzoyl)-5-bromo-4-chloro-2-ethoxypyridine | Pale yellow oily substance |
| 1-8 | 3-(2,3,4-trimethoxy-6-methylbenzoyl)-4-chloro-5-iodo-2-methoxypyridine | colorless oily substance |
| 1-9 | 3-(2,3,4-trimethoxy-6-methylbenzoyl)-5-iodo-2,4-dimethoxypyridine | colorless oily substance |
| 1-10 | 3-(2,3,4-trimethoxy-6-methylbenzoyl)-5-chloro-2-methoxy-4-methylthiopyridine | m.p. 98-104°C |
| 1-11 | 3-(2,3,4-trimethoxy-6-methylbenzoyl)-5-chloro-2,4-dimethoxypyridine | m.p. 93-94°C |
| 1-12 | 3-(2,3,4-trimethoxy-6-methylbenzoyl)-4,5-dibromo-2-methoxypyridine | m.p. 149-150°C |
| 1-13 | 3-(2,3,4-trimethoxy-6-methylbenzoyl)-4-bromo-2-methoxy-5-methylpyridine | m.p. 140-142°C |
| 1-14 | 3-(2,3,4-trimethoxy-6-methylbenzoyl)-5-bromo-4-trifluoromethyl-2-methoxypyridine | m.p. 128-129°C |
| 1-15 | 3-(2,3,4-trimethoxy-6-methylbenzoyl)-4,5-dichloro-2-methoxypyridine | m.p. 84-86°C |
| 1-16 | 3-(2,3,4-trimethoxy-6-methylbenzoyl)-2,4-dichloro-5-methylpyridine | m.p. 78-80°C |
| 1-17 | 3-(2,3,4-trimethoxy-6-methylbenzoyl)-2,4-dichloro-5-iodopyridine | Oily substance |
| 1-18 | 3-(2,3,4-trimethoxy-6-methylbenzoyl)-2-fluoro-4-iodo-5-methylpyridine | m.p. 140-141°C |
| 1-19 | 3-(2,3,4-trimethoxy-6-methylbenzoyl)-2-fluoro-4,5-dimethylpyridine | m.p. 91-92°C |
| 1-20 | 3-(2,3,4-trimethoxy-6-methylbenzoyl)-2-methoxy-4,5-dimethylpyridine | m.p. 84-86°C |
| 1-21 | 3-(2-ethoxy-3,4-dimethoxy-6-methylbenzoyl)-2-ethoxy-4,5-dimethylpyridine | m.p. 74-75°C |
| 1-22 | 3-(2,3,4-trimethoxy-6-methylbenzoyl)-4,5-dimethyl-2-methylthiopyridine | Oily substance |
| 1-23 | 3-(2,3,4-trimethoxy-6-methylbenzoyl)-5-bromo-4-chloro-2-methoxypyridine | m.p. 84-87°C |
| 1-24 | 3-(2,3,4-trimethoxy-6-methylbenzoyl)-4-chloro-2-methoxy-5-methylpyridine | m.p. 88-90°C |
| 1-25 | 3-(2,3,4-trimethoxy-6-methylbenzoyl)-2-chloro-5-trifluoromethyl-4-methylpyridine | |
| 1-26 | 3-(2,3,4-trimethoxy-6-methylbenzoyl)-5-trifluoromethyl-2-methoxy-4-methylpyridine | |
| 1-27 | 3-(2,3,4-trimethoxy-6-methylbenzoyl)-2,4-dichloro-5-trifluoromethylpyridine | |
| 1-28 | 3-(2,3,4-trimethoxy-6-methylbenzoyl)-4-chloro-5-trifluoromethyl-2-methoxypyridine | |
| 1-29 | 3-(2,3,4-trimethoxy-6-methylbenzoyl)-5-chloro-4-ethynyl-2-methoxypyridine | m.p. 113-115°C |
| 1-30 | 3-(2,3,4-trimethoxy-6-methylbenzoyl)-5-chloro-4-fluoromethyl-2-methoxypyridine | m.p. 82-84°C |
| 1-31 | 3-(2,3,4-trimethoxy-6-methylbenzoyl)-5-bromo-4-fluoromethyl-2-methoxypyridine | |
| 1-32 | 3-(2,3,4-trimethoxy-6-methylbenzoyl)-4-fluoromethyl-2-methoxy-5-methylpyridine | |
| 1-33 | 3-(2,3,4-trimethoxy-6-methylbenzoyl)-5-chloro-4-difluoromethyl-2-methoxypyridine | |
| 1-34 | 3-(2,3,4-trimethoxy-6-methylbenzoyl)-5-ethyl-4-trifluoromethyl-2-methoxypyridine | |
| 1-35 | 3-(2,3,4-trimethoxy-6-methylbenzoyl)-5-chloro-2-methoxy-4-methylpyridine | m.p. 84-88°C |
| 1-36 | 3-(2,3,4-trimethoxy-6-methylbenzoyl)-5-bromo-2-methoxy-4-methylpyridine | m.p. 111-114°C |
| 1-37 | 3-(2,3,4-trimethoxy-6-methylbenzoyl)-4-trifluoromethyl-2-methoxy-5-methylpyridine | m.p. 103-105°C |
| 1-38 | 3-(4,5-dimethoxy-2-methylbenzoyl)-5-chloro-2-methoxy-4-methylpyridine | m.p. 129-130°C |

In Table 1, Compound Nos. 1-1 to 1-34 and 1-38 are compounds not specifically disclosed in WO02/2527.

Compounds which are other than the above compounds and which are used in the following Test Examples, are shown in Table 2.

**TABLE 2**

| No. No. | Names of compounds Names of compounds | Physical properties |
|---|---|---|
| 2-1 | 4-(2,3,4-trimethoxy-6-methylbenzoyl)-2-chloro-5-methoxy-3-methylpyridine | m.p. 67-72°C |
| 2-2 | 4-(2,3,4-trimethoxy-6-methylbenzoyl)-3-trifluoromethyl-5-methoxy-2-methylpyridine | m.p. 137-138°C |
| 2-3 | 4-(2,3,4-trimethoxy-6-methylbenzoyl)-2-bromo-5-methoxy-3-methylpyridine | Pale yellow oily substance |
| 2-4 | 4-(2,3,4-trimethoxy-6-methylbenzoyl)-3-chloro-5-methoxy-2-methylpyridine | m.p. 102-103°C |
| 2-5 | 3-(2,3,4-trimethoxy-6-methylbenzoyl)-2,4-dimethoxypyridine | m.p. 74-75°C |
| 2-6 | 4-(2,3,4-trimethoxy-6-methylbenzoyl)-2,3-dichloro-5-methoxypyridine | m.p. 98-99°C |

Phenylpyridylmethanol derivatives of the present invention which are produced by methods in accordance with the above Synthesis Examples 1 to 4 and Synthesis Examples 1 to 16 in WO02/2527 will be shown in the following Table 3, and their NMR spectrum data will be shown in Table 4.

**TABLE 3**

| No. | Names of compounds |
|---|---|
| 3-1 | (2,3,4-trimethoxy-6-methylphenyl) (4-bromo-5-chloro-2-methoxy-3-pyridyl)methanol |
| 3-2 | (2,3,4-trimethoxy-6-methylphenyl)(5-chloro-4-ethyl-2-methoxy-3-pyridyl)methanol |
| 3-3 | (4,5-dimethoxy-2-methylphenyl)(4,5-dichloro-2-methoxy-3-pyridyl)methanol |
| 3-4 | (5-ethoxy-4-methoxy-2-methylphenyl)(4,5-dichloro-2-methoxy-3-pyridyl)methanol |
| 3-5 | (2,3,4-trimethoxy-6-methylphenyl)(4-bromo-5-chloro-2-ethoxy-3-pyridyl)methanol |
| 3-6 | (2,3,4-trimethoxy-6-methylphenyl) (5-chloro-2-ethoxy-4-methyl-3-pyridyl)methanol |
| 3-7 | (2,3,4-trimethoxy-6-methylphenyl) (5-bromo-4-chloro-2-ethoxy-3-pyridyl)methanol |
| 3-8 | (2,3,4-trimethoxy-6-methylphenyl)(4-chloro-5-iodo-2-methoxy-3-pyridyl)methanol |
| 3-9 | (2,3,4-trimethoxy-6-methylphenyl)(5-iodo-2,4-dimethoxy-3-pyridyl)methanol |
| 3-10 | (2,3,4-trimethoxy-6-methylphenyl)(5-chloro-2-methoxy-4-methylthio-3-pyridyl)methanol |
| 3-11 | (2,3,4-trimethoxy-6-methylphenyl)(5-chloro-2,4-dimethoxy-3-pyridyl)methanol |
| 3-12 | (2,3,4-trimethoxy-6-methylphenyl)(4,5-dibromo-2-methoxy-3-pyridyl)methanol |
| 3-13 | (2,3,4-trimethoxy-6-methylphenyl)(4-bromo-2-methoxy-5-methyl-3-pyridyl)methanol |
| 3-14 | (2,3,4-trimethoxy-6-methylphenyl)(5-bromo-4-trifluoromethyl-2-methoxy-3-pyridyl)methanol |
| 3-15 | (2,3,4-trimethoxy-6-methylphenyl)(4,5-dichloro-2-methoxy-3-pyridyl)methanol |
| 3-16 | (2,3,4-trimethoxy-6-methylphenyl)(2,4-dichloro-5-methyl-3-pyridyl)methanol |
| 3-17 | (2,3,4-trimethoxy-6-methylphenyl)(2,4-dichloro-5-iodo-3-pyridyl)methanol |
| 3-18 | (2,3,4-trimethoxy-6-methylphenyl)(2-fluoro-4-iodo-5-methyl-3-pyridyl)methanol |
| 3-19 | (2,3,4-trimethoxy-6-methylphenyl)(2-fluoro-4,5-dimethyl-3-pyridyl)methanol |
| 3-20 | (2,3,4-trimethoxy-6-methylphenyl)(2-methoxy-4,5-dimethyl-3-pyridyl)methanol |
| 3-21 | (2-ethoxy-3,4-dimethoxy-6-methylphenyl)(2-ethoxy-4,5-dimethyl-3-pyridyl)methanol |
| 3-22 | (2,3,4-trimethoxy-6-methylphenyl)(4,5-dimethyl-2-methylthio-3-pyridyl)methanol |
| 3-23 | (2,3,4-trimethoxy-6-methylphenyl)(5-bromo-4-chloro-2-methoxy-3-pyridyl)methanol |
| 3-24 | (2,3,4-trimethoxy-6-methylphenyl)(4-chloro-2-methoxy-5-methyl-3-pyridyl)methanol |
| 3-25 | (2,3,4-trimethoxy-6-methylphenyl)(2-chloro-5-trifluoromethyl-4-methyl-3-pyridyl)methanol |
| 3-26 | (2,3,4-trimethoxy-6-methylphenyl)(5-trifluoromethyl-2-methoxy-4-methyl-3-pyridyl)methanol |
| 3-27 | (2,3,4-trimethoxy-6-methylphenyl)(2,4-dichloro-5-trifluoromethyl-3-pyridyl)methanol |
| 3-28 | (2,3,4-trimethoxy-6-methylphenyl)(4-chloro-5-trifluoromethyl-2-methoxy-3-pyridyl)methanol |
| 3-29 | (2,3,4-trimethoxy-6-methylphenyl) (5-chloro-4-ethynyl-2-methoxy-3-pyridyl)methanol |
| 3-30 | (2,3,4-trimethoxy-6-methylphenyl)(5-chloro-4-fluoromethyl-2-methoxy-3-pyridyl)methanol |
| 3-31 | (2,3,4-trimethoxy-6-methylphenyl)(5-bromo-4-fluoromethyl-2-methoxy-3-pyridyl)methanol |
| 3-32 | (2,3,4-trimethoxy-6-methylphenyl)(4-fluoromethyl-2-methoxy-5-methyl-3-pyridyl)methanol |
| 3-33 | (2,3,4-trimethoxy-6-methylphenyl)(5-chloro-4-difluoromethyl-2-methoxy-3-pyridyl)methanol |
| 3-34 | (2,3,4-trimethoxy-6-methylphenyl)(5-ethyl-4-trifluoromethyl-2-methoxy-3-pyridyl)methanol |
| 3-35 | (2,3,4-trimethoxy-6-methylphenyl)(5-chloro-2-methoxy-4-methyl-3-pyridyl)methanol |
| 3-36 | (2,3,4-trimethoxy-6-methylphenyl)(5-bromo-2-methoxy-4-methyl-3-pyridyl)methanol |
| 3-37 | (2,3,4-trimethoxy-6-methylphenyl)(4-trifluoromethyl-2-methoxy-5-methyl-3-pyridyl)methanol |
| 3-38 | (4,5-dimethoxy-6-methylphenyl) (5-chloro-2-methoxy-4-methyl-3-pyridyl)methanol |

**TABLE 4**

| Compound No. | ¹H-NMR(CDCl₃, 400 MHz) : δ (ppm) |
|---|---|
| 3-1 | 2.33(s, 3H), 3.46(s, 3H), 3.77(s, 3H), 3.82(s, 3H), 3.94(s, 3H), 5.33(d, 1H, J = 10.4 Hz), 6.17(d, 1H, J = 10.4 Hz), 6.48(s, 1H), 8.06(s, 1H) |
| 3-3 | 2.42(s, 3H), 3.70(s, 3H), 3.77(d, 1H, J = 11.6 Hz), 3.86(s, 3H), 4.00(s, 3H), 6.32(d, 1H, J = 11.6 Hz), 6.58(s, 1H),6.72(s, 1H), 8.18(s, 1H) |
| 3-4 | 1.30(t, 3H, J = 6.8 Hz), 2.35(s, 3H), 3.74(d, 1H, J = 11.2 Hz), 3.78(s, 3H), 3.86(q, 2H, J = 6.8 Hz), 3.93(s, 3H), 6.24(d, 1H, J = 11.2 Hz), 6.50(s, 1H), 6.65(s, 1H), 8.11(s, 1H) |
| 3-5 | 1.41(t, 3H, J = 4.4 Hz), 2.24(s, 3H), 3.77(s, 3H), 3.82(s, 3H), 4.09(s, 3H), 4.44(q, 2H, J = 4.4 Hz), 5.36(d, 1H, J=9.6Hz), 6.34(d, 1H, J = 9.6 Hz), 6.48(s, 1H), 7.98(s, 1H) |
| 3-7 | 1.35(t, 3H, J = 4.4 Hz), 2.33(s, 3H), 3.49(s, 3H), 3.77(s, 3H), 3.83(s, 3H), 4.44(q, 2H, J = 4.4 Hz), 5.38(d, 1H, J = 10.0 Hz), 6.23(d, 1H, J = 10.0 Hz), 6.48(s, 1H), 8.17(s, 1H) |
| 3-8 | 2.34(s, 3H), 3.51(s, 3H), 3.77(s, 3H), 3.82(s, 3H), 3.95(s, 3H), 5.31(d, 1H, J = 10.4 Hz), 6.23(d, 1H, J = 10.4 Hz), 6.47(s, 1H), 8.36(s, 1H) |
| 3-12 | 2.24(s, 3H), 3.53(s, 3H), 3.77(s, 3H), 3.83(s, 3H), 3.97(s, 3H), 4.50-4.65(br, 1H), 6.28-6.34(br, 1H), 6.48(s, 1H), 8.16(s, 1H) |
| 3-14 | 2.35(s, 3H), 3.29(s, 3H), 3.74(s, 3H), 3.82(s, 3H), 3.90(s, 3H), 4.86-4.88(br, 1H), 6.19-6.22(br, 1H), 6.51(s, 1H), 8.31(s, 1H) |
| 3-15 | 2.32(s, 3H), 3.52(s, 3H), 3.77(s, 3H), 3.82(s, 3H), 4.11(s, 3H), 5.32(d, 1H,J = 10.0 Hz), 6.21(d, 1H, J = 10.0 Hz), 6.55(s, 1H), 8.07(s, 1H) |
| 3-16 | 2.10(s, 3H), 2.26(s, 3H), 3.59(s, 3H), 3.73(s, 3H), 3.77(s, 3H), 5.15(d, 1H, J = 9.2 Hz), 6.41(d, 1H, J = 9.2 Hz), 6.42(s, 1H), 8.08(s, 1H) |
| 3-17 | 2.18(s, 3H), 3.62(s, 3H), 3.76(s, 3H), 3.81(s, 3H), 5.02(brs, 1H), 6.41(brs, 1H), 6.46(s, 1H), 8.57(s, 1H) |
| 3-18 | 2.21(s, 3H), 2.42(s, 3H), 3.72(s, 3H), 3.79(s, 3H), 3.81(s, 3H), 4.97(d, 1H, J = 10.0 Hz), 6.07(d, 1H, J = 10.0 Hz), 6.46(s, 1H), 7.86(s, 1H) |
| 3-23 | 2.17(s, 3H), 2.33(s, 3H), 3.54(s, 3H), 3.79(s, 3H), 3.84(s, 3H), 3.97(s, 3H), 5.32(d, 1H, J = 10.0 Hz), 6.23(d, 1H, J = 10.0 Hz), 6.49(s, 1H), 8.21(s, 1H) |
| 3-30 | 2.32(s, 3H), 3.37(s, 3H), 3.75(s, 3H), 3.79(s, 3H), 3.81(s, 3H), 4.28(d, 1H, J = 6.0 Hz), 5.59(dd, 1H, J = 10.4 Hz, 46.8 Hz), 5.97(dd, 1H, J = 10.4 Hz, 46.8 Hz), 6.27(d, 1H, J = 6.0 Hz), 6.47(s, 1H), 8.08(s, 1H) |
| 3-35 | 2.26(s, 3H), 2.27(s, 3H), 3.54(s, 3H), 3.80(s, 3H), 3.84(s, 3H), 3.94(s, 3H), 5.32(d, 1H, J = 9.0 Hz), 6.12(d, 1H,J = 9.0 Hz), 6.47(s, 1H), 8.02(s, 1H) |
| 3-38 | 2.26(s, 3H), 2.38(s, 3H), 3.69(s, 3H), 3.85(br,1H), 3.87(s, 3H), 4.00(s, 3H), 6.13(s, 1H), 6.57(s, 1H), 6.72(s, 1H), 8.11(s, 1H) |

Now, Formulation Examples of the present invention will be described below. However, the formulation dose, the dosage form or the like is by no means restricted to the following Examples.

### FORMULATION EXAMPLE 1

| | |
|---|---|
| (1) Compound of the present invention | |
| | 20 parts by weight |
| (2) Clay | 72 parts by weight |
| (3) Sodium lignin sulfonate | 8 parts by weight |

The above components are uniformly mixed to obtain a wettable powder.

### FORMULATION EXAMPLE 2

| | |
|---|---|
| (1) Compound of the present invention | |
| | 5 parts by weight |
| (2) Talc | 95 parts by weight |

The above components are uniformly mixed to obtain a dust.

### FORMULATION EXAMPLE 3

| | |
|---|---|
| (1) Compound of the present invention | |
| | 20 parts by weight |
| (2) N,N'-dimethylacetamide | 20 parts by weight |
| (3) Polyoxyethylene alkyl phenyl ether | |
| | 10 parts by weight |
| (4) Xylene | 50 parts by weight |

The above components are uniformly mixed and dissolved to obtain an emulsifiable concentrate.

### FORMULATION EXAMPLE 4

| | |
|---|---|
| (1) Clay | 68 parts by weight |
| (2) Sodium lignin sulfonate | 2 parts by weight |
| (3) Polyoxyethylene alkyl aryl sulfate | |
| | 5 parts by weight |
| (4) Fine silica | 25 parts by weight |

A mixture of the above components and the compound of the present invention are mixed in a weight ratio of 4:1 to obtain a wettable powder.

### FORMULATION EXAMPLE 5

| | |
|---|---|
| (1) Compound of the present invention | 50 parts by weight |
| (2) Oxylated polyalkylphenyl phosphate triethanolamine | 2 parts by weight |
| (3) Silicone | 0.2 part by weight |
| (4) Water | 47.8 parts by weight |

The above components are uniformly mixed and pulverized to obtain a stock solution, and

| | |
|---|---|
| (5) Sodium polycarboxylate | 5 parts by weight |
| (6) Anhydrous sodium sulfate | 42.8 parts by weight |

are further added thereto, followed by uniform mixing, granulation and drying to obtain a granular wettable powder.

### FORMULATION EXAMPLE 6

| | |
|---|---|
| (1) Compound of the present invention | |
| | 5 parts by weight |
| (2) Polyoxyethylene octylphenyl ether | |
| | 1 part by weight |
| (3) Phosphate of polyoxyethylene | |
| | 0.1 part by weight |
| (4) Particulate calcium carbonate | |
| | 93.9 parts by weight |

The above components (1) to (3) are preliminarily mixed uniformly and diluted with a proper amount of acetone, the diluted mixture is sprayed on the component (4), and acetone is removed to obtain granules.

### FORMULATION EXAMPLE 7

| | |
|---|---|
| (1) Compound of the present invention | |
| | 2.5 parts by weight |
| (2) N-methyl-2-pyrrolidone | 2.5 parts by weight |
| (3) Soybean oil | 95.0 parts by weight |

The above components are uniformly mixed and dissolved to obtain an ultra low volume formulation.

### FORMULATION EXAMPLE 8

| | |
|---|---|
| (1) Compound of the present invention | |
| | 20 parts by weight |
| (2) Oxylated polyalkylphenol phosphate | |
| triethanolamine | 2 parts by weight |
| (3) Silicone | 0.2 part by weight |
| (4) Xanthan gum | 0.1 part by weight |
| (5) Ethylene glycol | 5 parts by weight |
| (6) Water | 72.7 parts by weight |

The above components are uniformly mixed and pulverized to obtain an aqueous suspension.

Now, Test Examples of the agricultural and horticultural fungicides will be described below.

### TEST EXAMPLE 1

### Tests on preventive effect against wheat powdery mildew

Wheat (cultivar: Norin-61-go) was cultivated in a polyethylene pot having a diameter of 7.5 cm, and when the wheat reached 1.5 leaf stage, the wheat was sprayed with 10 mL of a drug solution having a predetermined concentration of the compound of the present invention by a spray gun. After the drug solution dried, the wheat was inoculated by spreading with conidiospore of fungi of powdery mildew, and the wheat was kept in a thermostatic chamber at 20°C. From 6 to 8 days after the inoculation, the area of sporogony was examined. As a result, among the above compounds, compounds Nos. 1-1, 1-2, 1-4, 1-5, 1-6, 1-7, 1-8, 1-10, 1-11, 1-12, 1-13, 1-14, 1-15, 1-16, 1-17, 1-18, 1-19, 1-20, 1-21, 1-22, 1-23, 1-24, 1-35, 1-36, 1-37, 2-1, 2-2, 2-3, 2-4, 2-5 and 2-6 showed controlling effects of at least 90% as compared with non-treated cases, at a concentration of 125 ppm.

### TEST EXAMPLE 2

### Test on preventive effect against rice blast

Rice (cultivar: Nihonbare) was calculated in a polyethylene pot having a diameter of 7.5 cm, and when the rice reached 1.5 leaf stage, the rice was sprayed with 10 mL of a drug solution having a predetermined concentration of the compound of the present invention by a spray gun. After the drug solution dried, the rice was sprayed and inoculated with a conidiospore suspension of fungi of rice blast, and the rice was kept in an inoculation box at 20°C for 24 hours, and then kept in a thermostatic chamber at 20°C. From 6 to 11 days after the inoculation, the number of lesions was examined. As a result, among the above compounds, the compounds Nos. 1-1, 1-2, 1-15, 1-16, 1-17, 1-22, 1-23, 1-24, 1-35 and 2-3 showed controlling effects of at least 90% as compared with non-treated cases, at a concentration of 500 ppm.

### TEST EXAMPLE 3

### Test on preventive effect against cucumber powdery mildew

Cucumber (cultivar: Suyo) was cultivated in a polyethylene pot having a diameter of 7.5 cm, and when the cucumber reached 1.5 leaf stage, the cucumber was sprayed with a 10 mL of a drug solution having a predetermined concentration of the compound of the present invention by a spray gun. After the drug solution dried, the cucumber was sprayed and inoculated with a conidiospore suspension of fungi of powdery mildew, and the cucumber was kept in a thermostatic chamber at 20°C. From 6 to 11 days after the inoculation, the area of sporogony was examined. As a result, among the above compounds, compounds Nos. 1-1, 1-2, 1-4, 1-5, 1-7, 1-8, 1-10, 1-11, 1-12, 1-13, 1-14, 1-15, 1-16, 1-21, 1-22, 1-23, 1-24, 1-35, 1-36, 1-37, 2-1, 2-2, 2-3 and 2-6 showed controlling effects of at least 90% as compared with non-treated cases, at a concentration of 125 ppm.

With respect to compounds showing excellent preventive effects in the above tests, the effects were confirmed by a test method under the following conditions, taking into consideration a change in the effects due to rainfall, in addition to the above test methods.

Now, a Test Example of rainfastness of the agricultural and horticultural fungicides of the present invention will be described.

### TEST EXAMPLE 4

### Test on rainfastness of effect against cucumber powdery mildew

With respect to compounds which showed effects in the above Tests, a change in the effects due to rainfall was investigated assuming a situation closer to the practical application.

Cucumber (cultivar: Suyo) was cultivated in a polyethylene pot having a diameter of 7.5 cm, and when the cucumber reached 1.5 leaf stage, the cucumber was sprayed with a drug solution having a predetermined concentration of the compound of the present invention by a spray gun (corresponding to 1,000 L/ha). A day after the treatment, 10 mm/hr of artificial rainfall was applied for 1 hour. Together with seedlings not subjected to rainfall treatment (preventive), the cucumber was sprayed and inoculated with a conidiospore suspension of fungi of powdery mildew, and the cucumber was kept in a thermostatic chamber at 20°C. From 6 to 11 days after the inoculation, the number of lesions was examined, and the controlling rate was calculated by comparison with the non-treated cases. The results are shown in Table 5. Further, among 3-benzoyl-2,4,5-substituted pyridine derivatives of the present invention, those, of which no test results are shown in Table 5, would show similar test results, if subjected to the same test as Test Example 4.

**TABLE 5**

| Controlling rate of cucumber powdery mildew (%) | | |
|---|---|---|
| | 500 gai/ha | |
| Compound No. | Preventive | Rainfall |
| 1-1 | 100 | 100 |
| 1-12 | 100 | 68 |
| 1-14 | 100 | 100 |
| 1-15 | 100 | 100 |
| 1-23 | 100 | 100 |
| 1-24 | 100 | 85 |
| 1-35 | 100 | 100 |
| 1-37 | 100 | 100 |
| 2-1 | 100 | 0 |
| 2-2 | 100 | 35 |
| 2-3 | 100 | 0 |
| 2-4 | 92.5 | 35 |
| 2-5 | 95 | 0 |
| 2-6 | 90 | 0 |

### INDUSTRIAL APPLICABILITY

The specific 3-benzoyl-2,4,5-substituted pyridine derivatives of the present invention or salts thereof show excellent effects as active ingredients of fungicides, and they are capable of exhibiting excellent effects even in the case where crops have received rainfall after their application.

## Claims

1. A fungicide having rainfastness, which contains at least one 3-benzoyl-2,4,5-substituted pyridine derivative selected from the group consisting of 3-(2,3,4-trimethoxy-6-methylbenzoyl)-4-bromo-5-chloro-2-methoxypyridine, 3-(2,3,4-trimethoxy-6-methylbenzoyl)-5-chloro-4-ethyl-2-methoxypyridine, 3-(4,5-dimethoxy-2-methylbenzoyl)-4,5-dichloro-2-methoxypyridine, 3-(5-ethoxy-4-methoxy-2-methylbenzoyl)-4,5-dichloro-2-methoxypyridine, 3-(2,3,4-trimethoxy-6-methylbenzoyl)-4-bromo-5-chloro-2-ethoxypyridine, 3-(2,3,4-trimethoxy-6-methylbenzoyl)-5-chloro-2-ethoxy-4-methylpyridine, 3-(2,3,4-trimethoxy-6-methylbenzoyl)-5-bromo-4-chloro-2-ethoxypyridine, 3-(2,3,4-trimethoxy-6-methylbenzoyl)-4-chloro-5-iodo-2-methoxypyridine, 3-(2,3,4-trimethoxy-6-methylbenzoyl)-5-iodo-2,4-dimethoxypyridine, 3-(2,3,4-trimethoxy-6-methylbenzoyl)-5-chloro-2-methoxy-4-methylthiopyridine, 3-(2,3,4-trimethoxy-6-methylbenzoyl)-5-chloro-2,4-dimethoxypyridine, 3-(2,3,4-trimethoxy-6-methylbenzoyl)-4,5-dibromo-2-methoxypyridine, 3-(2,3,4-trimethoxy-6-methylbenzoyl)-4-bromo-2-methoxy-5-methylpyridine, 3-(2,3,4-trimethoxy-6-methylbenzoyl)-5-bromo-4-trifluoromethyl-2-methoxypyridine, 3-(2,3,4-trimethoxy-6-methylbenzoyl)-4,5-dichloro-2-methoxypyridine, 3-(2,3,4-trimethoxy-6-methylbenzoyl)-2,4-dichloro-5-methylpyridine, 3-(2,3,4-trimethoxy-6-methylbenzoyl)-2,4-dichloro-5-iodopyridine, 3-(2,3,4-trimethoxy-6-methylbenzoyl)-2-fluoro-4-iodo-5-methylpyridine, 3-(2,3,4-trimethoxy-6-methylbenzoyl)-2-fluoro-4,5-dimethylpyridine, 3-(2,3,4-trimethoxy-6-methylbenzoyl)-2-methoxy-4,5-dimethylpyridine, 3-(2-ethoxy-3,4-dimethoxy-6-methylbenzoyl)-2-ethoxy-4,5-dimethylpyridine, 3-(2,3,4-trimethoxy-6-methylbenzoyl)-4,5-dimethyl-2-methylthiopyridine, 3-(2,3,4-trimethoxy-6-methylbenzoyl)-5-bromo-4-chloro-2-methoxypyridine, 3-(2,3,4-trimethoxy-6-methylbenzoyl)-4-chloro-2-methoxy-5-methylpyridine, 3-(2,3,4-trimethoxy-6-methylbenzoyl)-2-chloro-5-trifluoromethyl-4-methylpyridine, 3-(2,3,4-trimethoxy-6-methylbenzoyl)-5-trifluoromethyl-2-methoxy-4-methylpyridine, 3-(2,3,4-trimethoxy-6-methylbenzoyl)-2,4-dichloro-5-trifluoromethylpyridine, 3-(2,3,4-trimethoxy-6-methylbenzoyl)-4-chloro-5-trifluoromethyl-2-methoxypyridine, 3-(2,3,4-trimethoxy-6-methylbenzoyl)-5-chloro-4-ethynyl-2-methoxypyridine, 3-(2,3,4-trimethoxy-6-methylbenzoyl)-5-chloro-4-fluoromethyl-2-methoxypyridine, 3-(2,3,4-trimethoxy-6-methylbenzoyl)-5-bromo-4-fluoromethyl-2-methoxypyridine, 3-(2,3,4-trimethoxy-6-methylbenzoyl)-4-fluoromethyl-2-methoxy-5-methylpyridine, 3-(2,3,4-trimethoxy-6-methylbenzoyl)-5-chloro-4-difluoromethyl-2-methoxypyridine, 3-(2,3,4-trimethoxy-6-methylbenzoyl)-5-ethyl-4-trifluoromethyl-2-methoxypyridine, 3-(2,3,4-trimethoxy-6-methylbenzoyl)-5-chloro-2-methoxy-4-methylpyridine, 3-(2,3,4-trimethoxy-6-methylbenzoyl)-5-bromo-2-methoxy-4-methylpyridine, 3-(2,3,4-trimethoxy-6-methylbenzoyl)-4-trifluoromethyl-2-methoxy-5-methylpyridine and 3-(4,5-dimethoxy-2-methylbenzoyl)-5-chloro-2-methoxy-4-methylpyridine, or a salt thereof, as an active ingredient.

2. At least one 3-benzoyl-2,4,5-substituted pyridine derivative selected from the group consisting of 3-(2,3,4-trimethoxy-6-methylbenzoyl)-4-bromo-5-chloro-2-methoxypyridine, 3-(2,3,4-trimethoxy-6-methylbenzoyl)-5-chloro-4-ethyl-2-methoxypyridine, 3-(4,5-dimethoxy-2-methylbenzoyl)-4,5-dichloro-2-methoxypyridine, 3-(5-ethoxy-4-methoxy-2-methylbenzoyl)-4,5-dichloro-2-methoxypyridine, 3-(2,3,4-trimethoxy-6-methylbenzoyl)-4-bromo-5-chloro-2-ethoxypyridine, 3-(2,3,4-trimethoxy-6-methylbenzoyl)-5-chloro-2-ethoxy-4-methylpyridine, 3-(2,3,4-trimethoxy-6-methylbenzoyl)-5-bromo-4-chloro-2-ethoxypyridine, 3-(2,3,4-trimethoxy-6-methylbenzoyl)-4-chloro-5-iodo-2-methoxypyridine, 3-(2,3,4-trimethoxy-6-methylbenzoyl)-5-iodo-2,4-dimethoxypyridine, 3-(2,3,4-trimethoxy-6-methylbenzoyl)-5-chloro-2-methoxy-4-methylthiopyridine, 3-(2,3,4-trimethoxy-6-methylbenzoyl)-5-chloro-2,4-dimethoxypyridine, 3-(2,3,4-trimethoxy-6-methylbenzoyl)-4,5-dibromo-2-methoxypyridine, 3-(2,3,4-trimethoxy-6-methylbenzoyl)-4-bromo-2-methoxy-5-methylpyridine, 3-(2,3,4-trimethoxy-6-methylbenzoyl)-5-bromo-4-trifluoromethyl-2-methoxypyridine, 3-(2,3,4-trimethoxy-6-methylbenzoyl)-4,5-dichloro-2-methoxypyridine, 3-(2,3,4-trimethoxy-6-methylbenzoyl)-2,4-dichloro-5-methylpyridine, 3-(2,3,4-trimethoxy-6-methylbenzoyl)-2,4-dichloro-5-iodopyridine, 3-(2,3,4-trimethoxy-6-methylbenzoyl) -2-fluoro-4-iodo-5-methylpyridine, 3-(2,3,4-trimethoxy-6-methylbenzoyl)-2-fluoro-4,5-dimethylpyridine, 3-(2,3,4-trimethoxy-6-methylbenzoyl)-2-methoxy-4,5-dimethylpyridine, 3-(2-ethoxy-3,4-dimethoxy-6-methylbenzoyl)-2-ethoxy-4,5-dimethylpyridine, 3-(2,3,4-trimethoxy-6-methylbenzoyl)-4,5-dimethyl-2-methylthiopyridine, 3-(2,3,4-trimethoxy-6-methylbenzoyl)-5-bromo-4-chloro-2-methoxypyridine, 3-(2,3,4-trimethoxy-6-methylbenzoyl)-4-chloro-2-methoxy-5-methylpyridine, 3-(2,3,4-trimethoxy-6-methylbenzoyl)-2-chloro-5-trifluoromethyl-4-methylpyridine, 3-(2,3,4-trimethoxy-6-methylbenzoyl)-5-trifluoromethyl-2-methoxy-4-methylpyridine, 3-(2,3,4-trimethoxy-6-methylbenzoyl)-2,4-dichloro-5-trifluoromethylpyridine, 3-(2,3,4-trimethoxy-6-methylbenzoyl)-4-chloro-5-trifluoromethyl-2-methoxypyridine, 3-(2,3,4-trimethoxy-6-methylbenzoyl)-5-chloro-4-ethynyl-2-methoxypyridine, 3-(2,3,4-trimethoxy-6-methylbenzoyl)-5-chloro-4-fluoromethyl-2-methoxypyridine, 3-(2,3,4-trimethoxy-6-methylbenzoyl)-5-bromo-4-fluoromethyl-2-methoxypyridine, 3-(2,3,4-trimethoxy-6-methylbenzoyl)-4-fluoromethyl-2-methoxy-5-methylpyridine, 3-(2,3,4-trimethoxy-6-methylbenzoyl)-5-chloro-4-difluoromethyl-2-methoxypyridine, 3-(2,3,4-trimethoxy-6-methylbenzoyl)-5-ethyl-4-trifluoromethyl-2-methoxypyridine and 3-(4,5-dimethoxy-2-methylbenzoyl)-5-chloro-2-methoxy-4-methylpyridine, or a salt thereof.

3. At least one phenylpyridylmethanol derivative selected from the group consisting of (2,3,4-trimethoxy-6-methylphenyl)(4-bromo-5-chloro-2-methoxy-3-pyridyl)methanol, (2,3,4-trimethoxy-6-methylphenyl)(5-chloro-4-ethyl-2-methoxy-3-pyridyl)methanol, (4,5-dimethoxy-2-methylphenyl)(4,5-dichloro-2-methoxy-3-pyridyl)methanol, (5-ethoxy-4-methoxy-2-methylphenyl)(4,5-dichloro-2-methoxy-3-pyridyl)methanol, (2,3,4-trimethoxy-6-methylphenyl)(4-bromo-5-chloro-2-ethoxy-3-pyridyl)methanol, (2,3,4-trimethoxy-6-methylphenyl)(5-chloro-2-ethoxy-4-methyl-3-pyridyl)methanol, (2,3,4-trimethoxy-6-methylphenyl)(5-bromo-4-chloro-2-ethoxy-3-pyridyl)methanol, (2,3,4-trimethoxy-6-methylphenyl)(4- chloro-5-iodo-2-methoxy-3-pyridyl)methanol, (2,3,4-trimethoxy-6-methylphenyl)(5-iodo-2,4-dimethoxy-3-pyridyl)methanol, (2,3,4-trimethoxy-6-methylphenyl)(5-chloro-2-methoxy-4-methylthio-3-pyridyl)methanol, (2,3,4-trimethoxy-6-methylphenyl)(5-chloro-2,4-dimethoxy-3-pyridyl)methanol, (2,3,4-trimethoxy-6-methylphenyl)(4,5-dibromo-2-methoxy-3-pyridyl)methanol, (2,3,4-trimethoxy-6-methylphenyl)(4-bromo-2-methoxy-5-methyl-3-pyridyl)methanol, (2,3,4-trimethoxy-6-methylphenyl)(5-bromo-4-trifluoromethyl-2-methoxy-3-pyridyl)methanol, (2,3,4-trimethoxy-6-methylphenyl)(4,5-dichloro-2-methoxy-3-pyridyl)methanol, (2,3,4-trimethoxy-6-methylphenyl)(2,4-dichloro-5-methyl-3-pyridyl)methanol, (2,3,4-trimethoxy-6-methylphenyl)(2,4-dichloro-5-iodo-3-pyridyl)methanol, (2,3,4-trimethoxy-6-methylphenyl)(2-fluoro-4-iodo-5-methyl-3-pyridyl)methanol, (2,3,4-trimethoxy-6-methylphenyl)(2-fluoro-4,5-dimethyl-3-pyridyl)methanol, (2,3,4-trimethoxy-6-methylphenyl)(2-methoxy-4,5-dimethyl-3-pyridyl)methanol, (2-ethoxy-3,4-dimethoxy-6-methylphenyl)(2-ethoxy-4,5-dimethyl-3-pyridyl)methanol, (2,3,4-trimethoxy-6-methylphenyl)(4,5-dimethyl-2-methylthio-3-pyridyl)methanol, (2,3,4-trimethoxy-6-methylphenyl)(5-bromo-4-chloro-2-methoxy-3-pyridyl)methanol, (2,3,4-trimethoxy-6-methylphenyl)(4-chloro-2-methoxy-5-methyl-3-pyridyl)methanol, (2,3,4-trimethoxy-6-methylphenyl)(2-chloro-5-trifluoromethyl-4-methyl-3-pyridyl)methanol, (2,3,4-trimethoxy-6-methylphenyl) (5-trifluoromethyl-2-methoxy-4-methyl-3-pyridyl)methanol, (2,3,4-trimethoxy-6-methylphenyl)(2,4-dichloro-5-trifluoromethyl-3-pyridyl)methanol, (2,3,4-trimethoxy-6-methylphenyl)(4-chloro-5-trifluoromethyl-2-methoxy-3-pyridyl)methanol, (2,3,4-trimethoxy-6-methylphenyl)(5-chloro-4-ethynyl-2-methoxy-3-pyridyl)methanol, (2,3,4-trimethoxy-6-methylphenyl)(5-chloro-4-fluoromethyl-2-methoxy-3-pyridyl)methanol, (2,3,4-trimethoxy-6-methylphenyl)(5-bromo-4-fluoromethyl-2-methoxy-3-pyridyl)methanol, (2,3,4,-trimethoxy-6-methylphenyl)(4-fluoromethyl-2-methoxy-5-methyl-3-pyridyl)methanol, (2,3,4-trimethoxy-6-methylphenyl)(5-chloro-4-difluoromethyl-2-methoxy-3-pyridyl)methanol, (2,3,4-trimethoxy-6-methylphenyl)(5-ethyl-4-trifluoromethyl-2-methoxy-3-pyridyl)methanol, (2,3,4-trimethoxy-6-methylphenyl) (5-chloro-2-methoxy-4-methyl-3-pyridyl)methanol, (2,3,4-trimethoxy-6-methylphenyl)(5-bromo-2-methoxy-4-methyl-3-pyridyl)methanol, (2,3,4-trimethoxy-6-methylphenyl)(4-trifluoromethyl-2-methoxy-5-methyl-3-pyridyl)methanol and (4,5-dimethoxy-6-methylphenyl)(5-chloro-2-methoxy-4-methyl-3-pyridyl)methanol, or a salt thereof.
